# EUROPÄISCHE PATENTSCHRIFT

(11) **EP 0 783 535 B1**
(45) Veröffentlichungstag und Bekanntmachung des Hinweises auf die Patenterteilung: **13.05.1998**
(21) Anmeldenummer: 95933397.2
(22) Anmeldetag: 19.09.1995
(51) Int. Cl.: C08F 26/06

(54) **VERFAHREN ZUR HERSTELLUNG WÄSSRIGER LÖSUNGEN VON POLY(N-VINYL-EPSILON-CAPROLACTAM) UND IHRE VERWENDUNG**
PROCESS FOR PREPARING AQUEOUS SOLUTIONS OF POLY(N-VINYL-EPSILON-CAPROLACTAM) AND THEIR USE
PROCEDE DE PREPARATION DE SOLUTIONS AQUEUSES DE POLY(N-VINYL-EPSILON-CAPROLACTAM) ET LEUR UTILISATION

(30) Priorität: 30.09.1994 DE 4434986
(43) Veröffentlichungstag der Anmeldung: 16.07.1997
(73) Patentinhaber: BASF AKTIENGESELLSCHAFT, 67056 Ludwigshafen (DE)
(72) Erfinder: KROKER, Jörg, D-67433 Neustadt (DE); SCHNEIDER, Reinhard, D-67136 Fussgönheim (DE); SCHUPP, Eberhard, D-67269 Grünstadt (DE); KERBER, Michael, D-69469 Weinheim (DE)
(86) Internationale Anmeldenummer: EP9503688
(87) Internationale Veröffentlichungsnummer: WO9610593

(56) Entgegenhaltungen:
- EP-A- 0 526 800
- KHIM. ATSETILENA, 1968 MOSCOW SU, Seiten 382-5, SIDEL'KOVSKAYA, IBRAMIGOV, ASKAROV 'synthesis of graft copolymers of cellulose and poly(n-vinyllactams)'

## Beschreibung

Die Erfindung betrifft ein Verfahren zur Herstellung wäßriger Lösungen von Poly(N-vinyl-ε-caprolactam) durch Polymerisieren von N-Vinyl-ε-caprolactam in wäßrigem Medium in Gegenwart von Polymerisationsinitiatoren.

N-Vinyl-ε-caprolactam kann beispielsweise in einem organischen Lösemittel oder auch in Wasser in Gegenwart von radikalbildenden Polymerisationsinitiatoren polymerisiert werden. Die Polymerisation von N-Vinylcaprolactam in Substanz kommt in der Technik praktisch nicht in Betracht, weil die Polymerisationsreaktion nur schlecht kontrollierbar ist und weil die Viskosität der Reaktionsmasse mit fortschreitender Polymerisation rasch ansteigt, so daß eine gute Durchmischung nicht mehr gewährleistet ist. Die Polymerisation von N-Vinylcaprolactam in einem organischen Lösemittel ist zwar technisch möglich, jedoch müssen die organischen Lösemittel für die weitere Verarbeitung des Polymeren abgetrennt werden. Aus anwendungstechnischer Sicht sind wäßrige Lösungen von Poly(N-vinyl-ε-caprolactam) von besonderem Interesse. Die Polymerisation von N-Vinyl-ε-caprolactam in Wasser gestaltet sich jedoch wegen des thermoreversiblen Lösungsverhaltens des Monomeren als kompliziert. Vinylcaprolactam hat einen Schmelzpunkt von 34°C und ist in kaltem Wasser praktisch unlöslich, während das Polymere in kaltem Wasser gut löslich, jedoch oberhalb der unteren kritischen Lösungstemperatur von ca. 35°C darin unlöslich ist. Ein naheliegender Kompromiß für die Polymerisation wäre die Durchführung der Reaktion bei einer Temperatur oberhalb des Schmelzpunkts von N-Vinylcaprolactam nach Art einer Öl-in-Wasser-Emulsionspolymerisation. Um daraus wäßrige Polyvinylcaprolactamlösungen herzustellen, muß man - insbesondere bei angestrebten Polymergehalten in der wäßrigen Lösung von mehr als 10 Gew.-% - das bei der Öl-in-Wasser-Emulsionspolymerisation erhaltene Reaktionsgemisch zusammen mit Wasser mehrere Stunden bis Tage rühren, bis aus der zähen bis glasartigen von Wasser umgebenen Polymermasse eine homogene gelkörperfreie Lösung erhalten wird.

Aus Makromol. Chem., Band 191, 169-184 (1990) ist ein Mikroemulsionsverfahren zur Polymerisation von N-Vinyl-ε-caprolactam in Wasser in Gegenwart von ca. 20 bis 50 Gew.-%, bezogen auf das Monomere, eines Sulfobernsteinsäuredialkylesters als Emulgator bekannt. Der Nachteil solcher Mikroemulsionen ist in dem hohen Emulgatorgehalt zu sehen.

Poly(N-vinyl-ε-caprolactam) wird beispielsweise als Polymeradditiv in Schmierstoffzusammensetzungen, als Schutzkolloid und als Binder für Non-Wovens eingesetzt, vgl. Ullmanns Encyclopedia of Industrial Chemistry, Band A 21, Seite 754 (1992). Aus der DE-B-1 240 812 und der DE-A-2 039 079 ist die Verwendung von Poly(N-vinylcaprolactam) und von Mischungen aus Polyvinylcaprolactam und wenigstens einem Alkalimetall- oder Ammoniumsalz eines Copolymerisates aus mindestens einem Acrylsäureester, einer α,β-monoethylenisch ungesättigten Carbonsäure und Methylmethacrylat als Klebemittel für Textilgut beim Filmdruck bekannt. Gemäß der Lehre der US-A-5 126 124 wird Polyvinylcaprolactam in Haarsprays verwendet. Die Tatsache, daß Polyvinylcaprolactam ein in Wasser thermoreversibles Lösungsverhalten mit einer unteren kritischen Lösungstemperatur von etwa 35°C aufweist, macht den Einsatz dieses Polymers als Opazifizierer in automatischen Abschattungssystemen interessant, vgl. DE-A-1 285 124.

Aus der EP-A-0 526 800 sind Polymerisate aus ethylenisch ungesättigten Verbindungen, die mindestens ein kovalent gebundenes N-Atom im Molekül enthalten, bekannt. Sie werden durch radikalische Polymerisation der Monomeren in Gegenwart von Monosacchariden, Oligosacchariden, Polysacchariden oder deren Derivaten in wäßrigen Systemen hergestellt. Die Polymerisate eignen sich als filmbildende Konditioniermittel in kosmetischen Zubereitungen und als Stabilisatoren für Parfüme und Parfümöle.

Der Erfindung liegt die Aufgabe zugrunde, ein Verfahren zur Herstellung von wäßrigen Lösungen von Polyvinylcaprolactam zur Verfügung zu stellen, wobei man relativ rasch homogene wäßrige Polymerlösungen erhält.

Die Aufgabe wird erfindungsgemäß gelöst mit einem Verfahren zur Herstellung wäßriger Lösungen von Poly(N-vinyl-ε-caprolactam) durch Polymerisieren von N-Vinyl-E-caprolactam in wäßrigem Medium in Gegenwart von Polymerisationsinitiatoren, wenn man die Polymerisation in Gegenwart von 0,1 bis 20 Gew.-%, bezogen auf die eingesetzten Monomeren, eines wasserlöslichen, synthetischen polymeren Schutzkolloids aus der Gruppe Polyvinylalkohol, teilverseiftes Polyvinylacetat, Polyvinylpyrrolidon, Polyacrylamid, Polymethacrylamid, Carboxylatgruppen enthaltende Polymerisate, Polyalkylvinylether und Mischungen der genannten Polymeren durchführt.

Bei der erfindungsgemäßen Polymerisation entstehen zunächst Suspensionen von feinverteilten Polymeren in dem wäßrigen Medium. Solche Suspensionen haben gegenüber wäßrigen Lösungen von Polyvinylcaprolactam bei gleichem Polymergehalt eine wesentlich geringere Viskosität. Sie sind leicht technisch handhabbar, d.h. sie können gut durchmischt und leicht mit Wasser zu gebrauchsfertigen Lösungen verdünnt werden, die beispielsweise Konzentrationen von 0,1 bis 50, vorzugsweise 1 bis 30 Gew.-% haben. Vorzugsweise stellt man die gebrauchsfertigen wäßrigen Lösungen in der Weise her, daß man die zum Verdünnen erforderliche Menge an Wasser unter Rühren zu einer Polyvinylcaprolactamlösung oder Suspension gibt, deren Temperatur oberhalb der unteren kritischen Lösungstemperatur der Polymeren liegt. Unabhängig davon kann man die gebrauchsfertigen Lösungen auch direkt durch Polymerisieren von Monomerlösungen herstellen, deren Konzentration der der Polymerlösungen entspricht.

N-Vinyl-ε-caprolactam ist beispielsweise durch Vinylierung von Caprolactam erhältlich.

Als Schutzkolloid für das erfindungsgemäße Verfahren eignen sich beispielsweise wasserlösliche synthetische Polymere. Beispiele für solche Polymere sind Polyvinylalkohol, teilverseiftes Polyvinylacetat, Polyvinylpyrrolidon, Polyacrylamid, Polymethacrylamid, Carboxylatgruppen enthaltende Polymerisate, Polyalkylvinylether und Mischungen der genannten Polymeren. Carboxylatgruppen enthaltende Polymerisate sind beispielsweise die aus Ammonium- und Alkalimetallsalzen von monoethylenisch ungesättigten C₃-C₅-Carbonsäuren wie Acrylsäure, Methacrylsäure, Maleinsäure und Itakonsäure. Hierzu gehören auch Copolymerisate der monoethylenisch ungesättigten Carbonsäuren untereinander als auch Copolymerisate von monoethylenisch ungesättigten Carbonsäuren mit anderen, damit copolymerisierbaren Monomeren, wie Acrylsäureestern, Methacrylsäureestern, Maleinsäureestern, wobei sich die Ester von Alkoholen mit C₁-C₁₈-Kohlenstoffatomen ableiten, z.B. Acrylsäuremethylester, Acrylsäureethylester, Acrylsäureisopropylester, Acrylsäure-n-propylester, Acrylsäureisobutylester, Acrylsäure-n-butylester, 2-Ethylhexylacrylat, Decylacrylat, Dodecylacrylat und Stearylacrylat, Methylmethacrylat, Methacrylsäure, Ethylester und Methacrylsäurebutylester. Weitere geeignete Comonomere sind beispielsweise Acrylamid, Methacrylamid, Vinylacetat, Vinylpropionat, Vinylbutyrat, Acrylnitril, Methacrylnitril und Hydroxyacrylester von monoethylenisch ungesättigten Carbonsäuren, wie Hydroxyethylacrylat, Hydroxymethacrylat, Hydroxypropylacrylat, Hydroxypropylmethacrylat, Hydroxybutylacrylat und Hydroxymethacrylat. Die Copolymerisate von monoethylenisch ungesättigten Carbonsäuren können mit 2 oder auch mehreren der genannten copolymerisierbaren Monomeren modifiziert werden. Die Carboxylatgruppen enthaltenen Polymerisate haben beispielsweise einen Carboxylatgruppenanteil von 5 bis 100 mol-%.

Besonders bevorzugt verwendet man als synthetische polymere Schutzkolloide Polyvinylalkohol und/oder teilverseiftes Polyvinylacetat mit einem Verseifungsgrad von 50 bis 99,9 mol-%.

Die polymeren Schutzkolloide, die erfindungsgemäß eingesetzt werden, werden dann als wasserlöslich bezeichnet, wenn sie in jedem Verhältnis in Wasser mischbar sind oder sich zu mindestens 0,1 Gew.-% in Wasser bei 20°C lösen und aus diesen wäßrigen Lösungen beim Verdünnen mit Wasser gleicher Temperatur nicht ausfallen. Das Molekulargewicht der wasserlöslichen, synthetischen polymeren Schutzkolloide beträgt beispielsweise 10 000 bis 2 000 000, vorzugsweise 25 000 bis 1 500 000. Die Viskosität der wäßrigen Lösungen der Schutzkolloide beträgt bei einer Konzentration der wäßrigen Lösung von 4 bis 10 Gew.-% und einer Temperatur von 20°C, beispielsweise 1 bis 10 000 mPas.

Die Mengen an wasserlöslichen, polymeren Schutzkolloiden, die bei dem erfindungsgemäßen Verfahren eingesetzt werden, betragen 0,1 bis 20, vorzugsweise 1 bis 5 Gew.-%, bezogen auf das bei der Polymerisation eingesetzte N-Vinyl-ε-caprolactam.

Die Polymerisation des Vinylcaprolactams erfolgt in wäßrigem Medium, wobei die Monomerkonzentration bis etwa 90 Gew.-% betragen kann und vorzugsweise in dem Bereich von 10 bis 60 Gew.-% liegt. Die Polymerisation wird vorzugsweise in reinem Wasser durchgeführt, kann jedoch auch in einem wäßrigem Medium vorgenommen werden, das bis zu 50 Gew.-% eines wasserlöslichen organischen Lösemittels enthält. Bei diesen Lösemitteln handelt es sich vorzugsweise um wasserlösliche, die Polymerisation regelnde Lösemittel, beispielsweise Ether wie Tetrahydrofuran oder Dioxan, Alkohole wie Methanol, Ethanol, Isopropanol und Ethylenglykol oder Glykolether wie Diethylenglykol oder Diethylenglykoldimethylether. Falls man ein regelndes wasserlösliches Lösemittel mitverwendet, beträgt die angewendete Menge beispielsweise 5 bis 50 Gew.-%, bezogen auf das eingesetzte Monomere.

Bei der Polymerisation können gegebenenfalls außer einem regelnd wirkenden organischen Lösemittel übliche Polymerisationsregler mitverwendet werden, z.B. regelnd wirkende Verbindungen, die Schwefel in gebundener Form enthalten wie Mercaptane, z.B. Mercaptoethanol, Mercaptopropanol, Mercaptobutanol, Dodecylmercaptan, Thioglycol, Mercaptoessigsäure und Mercaptopropionsäure. Außerdem eignen sich Allylverbindungen wie Allylalkohol und Butenole sowie Aldehyde wie Formaldehyd oder Acetaldehyd. Falls gewünscht, kann die Polymerisation auch in Gegenwart mehrerer Regler vorgenommen werden. Sofern man bei der Polymerisation einen Regler einsetzt, betragen die Einsatzmengen beispielsweise 0,05 bis 20, vorzugsweise 0,1 bis 10 Gew.-%, bezogen auf N-Vinylcaprolactam.

Als radikalbildende Initiatoren sind vorzugssweise alle diejenigen Verbindungen geeignet, die bei der gewählten Polymerisationstemperatur eine Halbwertzeit von weniger als 3 Stunden aufweisen. Man kann auch die Polymerisation zunächst bei niedrigerer Temperatur starten, um sie bei höherer Temperatur zu Ende zu führen. Es kann dann zweckmäßig sein, mit mindestens zwei bei verschiedenen Temperaturen zerfallenden Initiatoren zu arbeiten, derart, daß mit einem bei niedriger Temperatur zerfallenden Initiator die Polymerisation gestartet wird, um sie dann mit einem bei höherer Temperatur zerfallenden Initiator zu Ende zu führen. Bevorzugt werden in Wasser oder niedrigen Alkoholen wie Methanol, Ethanol und Isopropanol, lösliche Initiatoren verwendet, beispielsweise 2,2'-Azobis(4-methoxy-2,4-dimethylvaleronitril), 2,2'-Azobis(2,4-dimethylvaleronitril), 2,2'-Azobis(2-methylpropionsäurenitril), 4,4'-Azobis(4-cyanovaleriansäure), Dimethyl-2,2'-Azobis(2-methylpropionat), 1,1'-Azobis(1-cyclohexancarbonitril), 2,2'-Azobis (N,N'-dimethyleneisobutyramidin), (1-Phenylethyl)azodiphenylmethan, 1-[(1-Cyano-1-methylethyl)azo]formamid, 2,2'-Azobis(N,N'-dimethyleneisobutyramidin)dihydrochlorid oder 2,2'-Azobis(2-amidinopropan)dihydrochlorid, sowie Acetylcyclohexansulfonylperoxid, Diacetylperoxydicarbonat, Diisopropylperoxydicarbonat, t-Amylperneodecanoat, t-Butylperneodecanoat, Bis(4-chlorbenzoyl)peroxid, Bis(2,4-dichlorbenzoyl)peroxid, t-Butylperpivalat, Bis(3,5,5-trimethylhexanoyl)peroxid, Di-octanoylperoxid, Diisononanoylperoxid, Didecanoylperoxid, Dilauroylperoxid, Bis(2-methylbenzoyl)peroxid, Succinylperoxid, Diacetylperoxid, Dibenzoylperoxid, Di-2-ethylhexylperoxidicarbonat, Dicyclohexylperoxidicarbonat, t-Butyl-per-2-ethylhexanoat, t-Butylperisobutyrat und t-Butylpermaleinat. Die Auswahl des oder der am besten geeigneten Iniatoren ergibt sich letztendlich aus der Temperatur, bei der die Polymerisationsreaktion ausgeführt werden soll.

Weniger bevorzugt, aber auch einsetzbar sind Redoxinitiatorsysteme, bestehend z.B. aus Peroxidgruppen aufweisenden Initiatoren, die durch den Zusatz von Reduktionsmitteln wie Übergangsmetallsalzen, Natriumsulfit, Natriumbisulfit, Natriumformaldehydsulfoxylat und/oder Hydrazin oder organischen Verbindungen wie Benzoin, Dimethylanilin und Ascorbinsäure gespalten werden. Derartige Redoxinitiatorsysteme ermöglichen die Polymerisation bei Temperaturen, die deutlich unter der Zerfallstemperatur der Peroxidgruppen aufweisenden Initiatoren liegen.

Bezogen auf N-Vinyl-ε-caprolactam setzt man beispielsweise 500 bis 50000, vorzugsweise 1000 bis 20000 ppm eines Initiators oder einer Mischung mehrerer Initiatoren ein.

Die Polymerisation von Vinylcaprolactam kann kontinuierlich oder diskontinuierlich erfolgen. Bevorzugt ist die diskontinuierliche Polymerisation in Rührbehältern, die mit wirksamen Mischorganen wie Blatt-, Anker-, Impeller- oder Propellerrührern ausgestattet sind oder in anderen geeigneten Aggregaten wie Knetern. Die Polymerisation wird üblicherweise in einer Inertgasatmosphäre, z.B. Stickstoff, durchgeführt. Die Temperaturen bei der Polymerisation betragen etwa 40 bis 150, vorzugsweise 60 bis 100°C, wobei man bei Temperaturen, die oberhalb des Siedepunkts des Reaktionsgemisches liegen, die Reaktion in druckdicht verschlossenen Apparaturen unter erhöhtem Druck durchführt.

Man erhält nach dem erfindungsgemäßen Verfahren wäßrige Lösungen von Poly(N-vinyl-ε-caprolactam), die während der Polymerisation oder nach Abschluß der Polymerisation ohne Schwierigkeit mit Wasser zu homogenen wäßrigen Lösungen einer gewünschten Konzentration verdünnt werden können. Die Wasserzugabe kann dabei kontinuierlich, absatzweise oder auf einmal erfolgen. Während man gemäß dem Stand der Technik bei der Herstellung wäßriger Lösungen von Polyvinylcaprolactam selbst feinteiliges Polyvinylcaprolactam lange in Wasser rühren muß, erhält man nach dem erfindungsgemäßen Verfahren wäßrige Polymerlösungen, die durch Zugabe von Wasser rasch auf die gewünschte niedrigere Polymerkonzentration verdünnt werden können.

Durch geeignete Kombination von Monomerkonzentration, Polymerisationstemperatur, Schutzkolloid, Initiator und Regler sowie der Konzentrationen der Einsatzstoffe erhält man Poly(N-vinyl-ε-caprolactam) mit K-Werten von 10 bis 300, vorzugsweise 15 bis 200 (bestimmt nach H. Fikentscher an 1 gew.-%igen wäßrigen Lösungen bei 25°C). Aus den bei der Polymerisation erhältlichen wäßrigen Lösungen können nach dem Abkühlen auf Temperaturen unterhalb der unteren kritischen Lösungstemperatur für Polyvinylcaprolactam durch Zugabe von Wasser rasch homogene gallert- und gelkörperfreie wäßrige Lösungen hergestellt werden.

Die wäßrigen Lösungen von Poly(N-vinyl-ε-caprolactam) werden beispielsweise als Textildruckkleber, als Klebronstoff, als Schmiermitteladditiv, als Hilfsmittel in kosmetischen Zubereitungen, als Waschmitteladditiv und als Opazifizierer in automatischen Abschattungssystemen verwendet. Kosmetische Zubereitungen umfassen beispielsweise Haarlack und Haarspray sowie hautkosmetische Zubereitungen. Die nach dem erfindungsgemäßen Verfahren erhältlichen wäßrigen Lösungen von Polyvinylcaprolactam können außerdem als Hilfsmittel im Bereich der Förderung, der Gewinnung und dem Transport von Erdöl und Erdgas, als Klärungsmittel der Getränkeindustrie, als Waschmitteladditiv als Hilfsmittel in der Agrochemie, z.B. als Strukturverbesserer für Ackerböden und zum Beschichten von Saatgut, zur Herstellung von Wirkstoffzubereitungen in pharmazeutischen oder Düngemittelformulierungen eingesetzt werden, die den Wirkstoff langsam abgeben, zur Reinigung von Abwässern sowie als Hilfsmittel in der Fotoindustrie.

Die Prozentangaben in den Beispielen bedeuten Gew.-%, die Teile sind Gewichtsteile. Die K-Werte der Polymerisate wurde nach H. Fikentscher, Cellulose-Chemie, Band 13, S. 58-63 und 71-74 (1932) in 1 %iger wäßriger Lösung bei 25°C ermittelt.

### Beispiele

In einer zylindrischen Rührapparatur, die mit einem Ankerrührer und Rückflußkühler ausgestattet war, wurden unter Stickstoffatmosphäre die in Tabelle 1 genannten Einsatzstoffe in den angegebenen Mengen vorgelegt und unter Rühren auf 70 bzw. 75°C erwärmt. Nachdem die daraufhin einsetzende exotherme Reaktion beendet war, wurde die Masse für 0,25 bis 2 h bei einer Temperatur des Heizbads von 90°C nachgerührt.

Im Fall der Vergleichsbeispiele lagen hochzähe bis glasartige Polymermassen vor, die von Wasser umgeben waren und die vom Ankerrührer nicht mehr durchmischt werden konnten. Bei den Beispielen erhielt man gut bewegliche, gut durchmischbare homogene Polymer-in-Wasser-Suspensionen.

Nach dem Entfernen des Heizbades erfolgt dann entweder innerhalb von 3 h die kontinuierliche Zugabe von kaltem Wasser zum Einstellen der gewünschten Polymerkonzentration oder lediglich ein Abkühlen der Reaktionsmischung auf Raumtemperatur innerhalb von 1 h. Bei den erfindungsgemäßen Beispielen war kein weiteres Nachrühren notwendig. Die Lösungen waren homogen und frei von jeglichen Gallert- und Gelkörpern. Die nach den Vergleichsbeispielen hergestellten Polymeren konnten nicht ohne weiteres mit Wasser verdünnt werden, sondern mußten längere Zeit gerührt werden, vgl. die in der Tabelle angegebenen Nachrührzeiten. Erst dann lagen bei den Vergleichsbeispielen homogene Lösungen vor. Bei den Vergleichsbeispielen 3 und 4, bei denen Sulfobernsteinsäurebis(2-ethylhexylester)Natriumsalz als Emulgator eingesetzt wurde, war ein erheblicner Teil an ungelöstem Polymer am Rührorgan verblieben.

Beispiel 4 und Vergleichsbeispiel 6 wurden jeweils in einem Laborkneter durchgeführt.

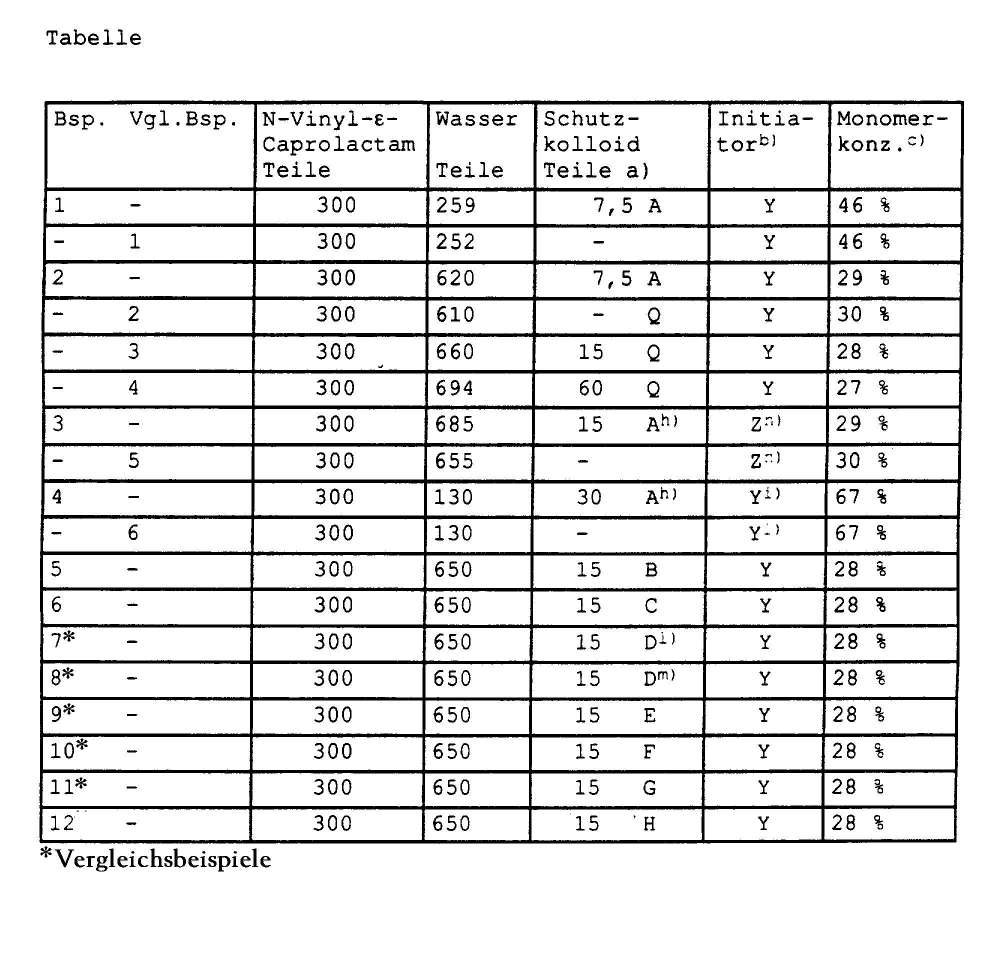

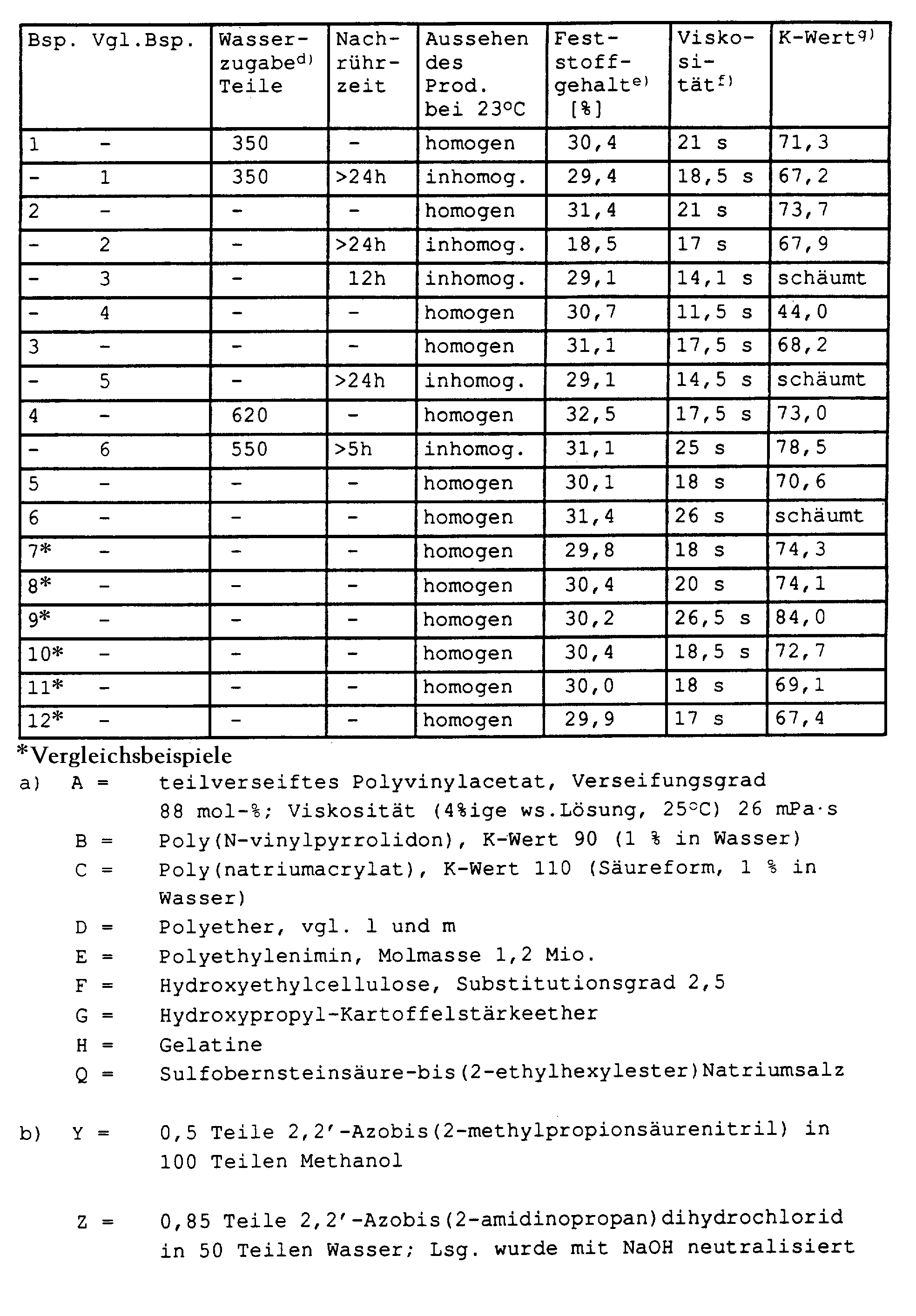

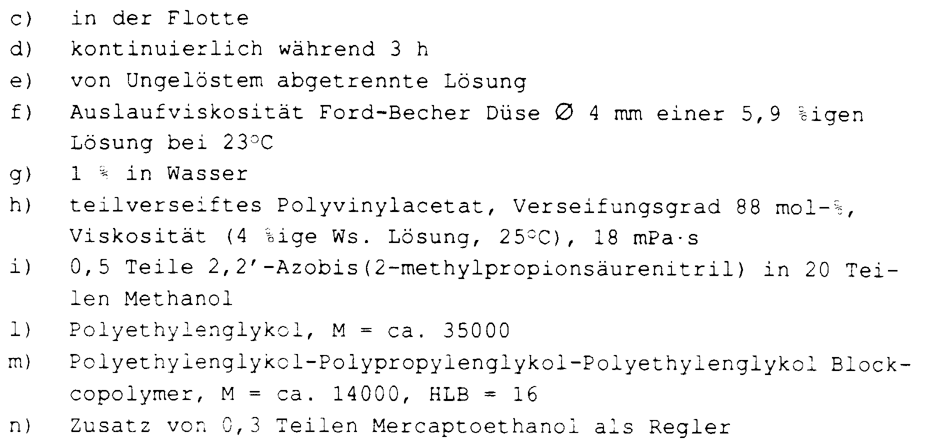

## Patentansprüche

1. Verfahren zur Herstellung wäßriger Lösungen von Poly(N-vinyl-ε-caprolactam) durch Polymerisieren von N-Vinyl-ε-caprolactam in wäßrigem Medium in Gegenwart von Polymerisationsinitiatoren, dadurch gekennzeichnet, daß man die Polymerisation in Gegenwart von 0,1 bis 20 Gew.-%, bezogen auf die eingesetzten Monomeren, eines wasserlöslichen, synthetischen polymeren Schutzkolloids aus der Gruppe Polyvinylalkohol, teilverseiftes Polyvinylacetat, Polyvinylpyrrolidon, Polyacrylamid, Polymethacrylamid, Carboxylatgruppen enthaltende Polymerisate, Polyalkylvinylether und Mischungen der genannten Polymeren durchführt.

2. Verfahren nach Anspruch 1, dadurch gekennzeichnet, daß man als wasserlösliche synthetische Polymere Polyvinylalkohol und/oder teilverseiftes Polyvinylacetat mit einem Verseifungsgrad von 50 bis 99,9 mol-% verwendet.

3. Verwendung der gemäß den Ansprüchen 1 bis 2 erhältlichen wäßrigen Lösungen von Poly(N-vinyl-ε-caprolactam) als Opazifizierer in automatischen Abschattungssystemen.

## Claims

1. A process for preparing an aqueous solution of poly(N-vinyl-ε-caprolactam) by polymerizing N-vinyl-ε-caprolactam in an aqueous medium in the presence of a polymerization initiator, which comprises polymerizing in the presence of from 0.1 to 20 % by weight, based on the monomer used, of a synthetic water-soluble polymeric protective colloid selected from the group consisting of polyvinyl alcohol, partially hydrolyzed polyvinyl acetate, polyvinylpyrrolidone, polyacrylamide, polymethacrylamide, carboxylate-functional addition polymers, polyalkyl vinyl ethers and mixtures thereof.

2. A process as claimed in claim 1, wherein the water-soluble synthetic polymer used is polyvinyl alcohol and/or partially hydrolyzed polyvinyl acetate having a degree of hydrolysis of from 50 to 99.9 mol%.

3. The use of the aqueous solution of poly(N-vinyl-ε-caprolactam) obtainable as claimed in claim 1 or 2 as an opacifier in automatic shading systems.

## Revendications

1. Procédé de préparation de solutions aqueuses de poly(N-vinyl-ε-caprolactame) par polymérisation de N-vinyl-ε-caprolactame en milieu aqueux en présence d'amorceurs de polymérisation, caractérisé en ce que l'on effectue la polymérisation en présence de 0,1 à 20% en poids, par rapport aux monomères introduits, d'un colloïde polymère de protection, soluble dans l'eau et synthétique, choisi dans le groupe formé par l'alcool polyvinylique, le poly(acétate de vinyle) partiellement saponifié, la polyvinylpyrrolidone, le poly(acrylamide), le poly(méthacrylamide), les polymères contenant des groupements carboxylate, les poly(alkylvinyléther)s et les mélanges des polymères mentionnés.

2. Procédé selon la revendication 1, caractérisé en ce que l'on utilise en tant que polymère synthétique soluble dans l'eau, de l'alcool polyvinylique et/ou du poly(acétate de vinyle) partiellement saponifié avec un taux de saponification de 50-99,9% en moles.

3. Utilisation des solutions aqueuses de poly(N-vinyl-ε-caprolactame) obtenues selon la revendication 1 ou 2 en tant qu'opacifiant pour des systèmes d'obscurcissement automatiques.
